# EUROPEAN PATENT SPECIFICATION

(11) **EP 2 131 680 B1**
(45) Date of publication and mention of the grant of the patent: **14.08.2013**
(21) Application number: 08735504.6
(22) Date of filing: 27.03.2008
(51) Int. Cl.: A23L 1/30, A61K 39/05

(54) **PROBIOTICS TO IMPROVE GUT MICROBIOTA**
PROBIOTIKA ZUR VERBESSERUNG DER DARMMIKROBIOTA
PROBIOTIQUES DESTINÉS À AMÉLIORER LA MICROFLORE INTESTINALE

(30) Priority: 28.03.2007 EP 07105074
(43) Date of publication of application: 16.12.2009
(73) Proprietor: Nestec S.A., 1800 Vevey (CH)
(72) Inventor: HUBER-HAAG, Karl-Josef, CH-1009 Pully (CH); FICHOT, Marie-Claire, CH-1807 Blonay (CH); ROCHAT, Florence, CH-1820 Montreux (CH); SPRENGER, Norbert, CH-1073 Savigny (CH)
(74) Representative: Corticchiato, Olivier
(86) International application number: PCT/EP2008/053611
(87) International publication number: WO 2008/116892

(56) References cited:
- EP-A- 0 768 375
- WO-A-2006/108824
- US-A1- 2003 129 278
- SALVINI F ET AL: "PROBIOTICS, PREBIOTICS AND CHILD HEALTH: WHERE ARE WE GOING?" JOURNAL OF INTERNATIONAL MEDICAL RESEARCH, CAMBRIDGE MEDICAL PUBLICATIONS LTD, GB, vol. 32, no. 2, 1 March 2004 (2004-03-01), pages 97-108, XP009041197 ISSN: 0300-0605
- GRÖNLUND M M ET AL: "FECAL MICROFLORA IN HEALTHY INFANTS BORN BY DIFFERENT METHODS OF DELIVERY: PERMANENT CHANGES IN INTESTINAL FLORA AFTER CESAREAN DELIVERY" JOURNAL OF PEDIATRIC GASTROENTEROLOGY AND NUTRITION, RAVEN PRESS, NEW YORK, NY, US, vol. 28, no. 1, 1 January 1999 (1999-01-01), pages 19-25, XP009084526 ISSN: 0277-2116
- AGOSTONI C ET AL: "PROBIOTIC BACTERIA IN DIETETIC PRODUCTS FOR INFANTS: A COMMENTARY BY THE ESPGHAN COMMITTEE ON NUTRITION" JOURNAL OF PEDIATRIC GASTROENTEROLOGY AND NUTRITION, RAVEN PRESS, NEW YORK, NY, US, vol. 38, no. 4, 1 April 2004 (2004-04-01), pages 365-374, XP009069353 ISSN: 0277-2116
- DOMINQUEZ ET AL.: "Delivery mode shapes the acquisition of the initial microbiota", PNAS, vol. 107, 29 June 2010 (2010-06-29), pages 11971-11975,
- J. NEU, J. RUSHING: "cesarian versus vaginal delivery", CLIN. PERINTOL., vol. 38, 1 January 2011 (2011-01-01), pages 321-331,

## Description

### Field of the Invention

This invention relates to the administration to infants delivered by Caesarean section of a specific probiotic bacterial strain capable of promoting an early bifidogenic gut microbiota.

### Background to the Invention

Immediately before birth, the gastro-intestinal tract of a baby is thought to be sterile. During the normal process of birth, it encounters bacteria from the digestive tract, skin and environment of the mother and starts to become colonised. The faecal microbiota of a healthy, vaginally-delivered, breast-fed infant of age 2 to 4 weeks which may be taken as the optimum microbiota for this age group is dominated by Bifidobacteria species with some Lactobacillus species and lesser amounts of Bacteroides such as *Bacteriodes fragilis* species, to the exclusion of potential pathogens such as Clostridia. After the completion of weaning at about 2 years of age, a pattern of gut microbiota that resembles the adult pattern becomes established.

It should be noted that, in the healthy, vaginally-delivered, breast-fed infant, Bifidobacteria form the basis of the microbiota accounting for 60-90 % of total bacteria in the infant gut. Breast feeding also promotes intestinal barrier development which, together with bifidobacterial domination leads to enhanced absorption and therefore utilisation of ingested nutrition.

Grönlund *et al* have studied the faecal microbiota of healthy infants born by caesarean section and compared it with that of a comparable group of infants born by vaginal delivery. They concluded that the gut flora of infants born by caesarean delivery may be disturbed for up to six months after the birth. Specifically they noted that the rates of colonisation by Bifidobacteria and Lactobacilli in the caesarean group reached the rates of colonisation in the vaginally delivered group only after one month and ten days respectively (Grönlund et al, "Fecal Microflora in Heathy Infants Born by Different Methods of Delivery: Permanent Changes in Intestinal Flora After Cesarean Delivery", Journal of Pediatric Gastroenterology and Nutrition, 28:19 - 25).

Other workers have suggested that this delayed/aberrant colonisation may have specific consequences in terms of the subsequent development of the infant and have linked these consequences to the differences in gut flora. For example, Laubereau *et al* found that infants born by caesarean section had a greater risk of diarrhoea than vaginally delivered infants (Laubereau et al, Caesarean Section and gastrointestinal symptoms, atopic dermatitis and sensitisation during the first year of life", Arch Dis Child 2004;89:993-997). Negele *et al* found that caesarean delivery may be an additional risk factor for wheezing and allergic sensitisation to food allergens up to the age of two years (Negele et al "Mode of delivery and development of atopic disease during the first 2 years of life" Pediatr Allergy Immunol 2004:15:48 - 54). It has also been suggested that systemic low-grade inflammation and a sub-optimal gut microbiota may also be implicated in the development of obesity (Fantuzzi G. "Adipose tissue, adipokines, and inflammation" J Allergy Clin Immunol. 2005;115:911-919, Bäckhed F, Ding H, Wang T, et al. "The gut microbiota as an environmental factor that regulates fat storage" Proc Natl Acad Sci USA. 2004;101:15718-15723).

Mother's milk is recommended for all infants. However, in some cases breast feeding is inadequate or unsuccessful for medical reasons or the mother chooses not to breast feed. Infant formulae have been developed for these situations.

In the recent past, certain strains of bacteria have attracted considerable attention because they have been found to exhibit valuable properties for man if ingested. In particular, specific strains of the genera Lactobacilli and Bifidobacteria have been found to be able to colonise the intestine, to reduce the capability of pathogenic bacteria to adhere to the intestinal epithelium, to have immunomodulatory effects and to assist in the maintenance of well-being. Such bacteria are sometimes called probiotics and it has already been proposed to add suitable probiotic bacteria to infant formulae.

Extensive studies have been carried out to identify new probiotic strains. For example, EP 0 199 535, EP 0 768 375, WO 97/00078, EP 0 577 903 and WO 00/53200 disclose specific strains of Lactobacilli and Bifidobacteria and their beneficial effects.

More recently, some concerns have been expressed about the addition of probiotic bacteria to infant formula which is intended as the sole source of nutrition for infants in the first six months of life. These concerns were summarized in the medical position paper from the ESPGHAN Committee on Nutrition entitled "Probiotic Bacteria in Dietetic Products for Infants" (Journal of Paediatric Gastroenterology and Nutrition, 38:365-374).

The intestinal microbiota plays an important role in the hydrolysis of indigestible oligosaccharides and polysaccharides to absorbable monosaccharides and activation of lipoprotein lipase by direct action on the villous epithelium. Further, it has recently been demonstrated that human milk contains not only oligosaccharides but also Bifidobacteria. At the same time, genomic studies have convincingly shown that Bifidobacteria present in the gut of breast-fed infants, such as *Bifidobacterium longum,* are specially equipped to utilize breast-milk oligosaccharides as nutrients. *Bifidobacterium longum* is also adapted to the conditions in the large intestine where energy harvest from slowly absorbable carbohydrates takes place.

In short, more and more evidence is emerging which suggests that the establishment of an appropriate intestinal microbiota early in life may be a significant in subsequent healthy development. At the same time the proportion of caesarean deliveries continues to increase reaching as much as 70% of all births in some countries. It is therefore clear that there is a need to provide a means to promote the rapid establishment of an appropriate intestinal microbiota in infants where this does not occur naturally. This need is particularly acute given the current practice of routinely administering prophylactic doses of antibiotics to pregnant women who undergo an elective caesarean delivery.

### Summary of the Invention

As noted above, in the healthy, vaginally-delivered, breast-fed infant, Bifidobacteria form the basis of the microbiota accounting for 60-90 % of total bacteria in the infant gut. The species of Bifidobacteria that are predominantly found in such infants are *Bifidobacterium breve, Bifidobacterium infantis,* and *Bifdobacterium longum.* The present inventors have surprisingly found that administration of a specific strain of a different species of Bifidobacteria, namely *Bifidobacterium lactis* CNCM I-3446 promotes the development of an early bifidogenic intestinal microbiota in infants delivered by caesarean section.

Accordingly the present invention provides the use of *Bifidobacterium lactis* CNCM I-3446 in the manufacture of a medicament or therapeutic nutritional composition for promoting the development of an early bifidogenic intestinal microbiota in infants delivered by caesarean section.

The invention further provides the use of *Bifdobacterium lactis* CNCM I-3446 in the manufacture of a medicament or therapeutic nutritional composition for reducing the risk of subsequent development of allergy in infants delivered by caesarean section.

In a further aspect, the invention provides the use of *Bifidobacterium lactis* CNCM I-3446 in the manufacture of a medicament or therapeutic nutritional composition for preventing or treating diarrhoea in infants delivered by caesarean section.

The invention extends to a method of promoting the development of an early bifidogenic intestinal microbiota in infants delivered by caesarean section comprising providing a therapeutic amount of *Bifidobacterium lactis* CNCM I-3446 to an infant born by caesarean section and in need of the same.

The invention further extends to a method of reducing the risk that an infant delivered by caesarean section will subsequently develop allergy comprising providing a therapeutic amount of *Bifidobacterium lactis* CNCM I-3446 to an infant born by caesarean section and in need of the same.

The invention also extends to a method of preventing or treating diarrhoea in an infant delivered by caesarean section comprising providing a therapeutic amount of *Bifidobacterium lactis* CNCM I-3446 to an infant born by caesarean section and in need of the same.

Without wishing to be bound by theory, the present inventors believe that administration of *Bifidobacterium lactis* CNCM I-3446 to an infant born by caesarean section in some way as yet incompletely understood primes the gastrointestinal tract of the infant to favour subsequent colonisation by those species of Bifidobacteria which are commonly found in the tracts of healthy, vaginally delivered infants. It is thought that this beneficial colonisation reduces the risk of episodes of diarrhoea such as have been shown to afflict infants delivered by caesarean section. It is further thought that the beneficial colonisation reduces the risk of subsequent development of allergy as manifested for example by wheezing and/or sensitisation to food allergens.

It should be noted that it is neither the object nor the effect of such treatment to promote colonisation by *Bifidobacterium lactis* CNCM I-3446 itself but rather to promote colonisation with other species so as to achieve an early bifidogenic intestinal microbiota comparable with that found in healthy, breast-fed, vaginally-delivered infants.

### Brief Description of the Drawings

**Figure 1** shows the *B. breve* and *B. longum* counts in faecal and jejunal content samples at day 14 of treatment in gnotobiotic mice gavaged with a human baby microbiota; and
**Figure 2** shows *C.perfringens* counts in jejunal content and faecal samples at day 14 of treatment in gnotobiotic mice gavaged with a human baby microbiota.

### Detailed Description of the Invention

In this specification, the following terms have the following meanings:-
"early bifidogenic intestinal microbiota" means for an infant up to the age of 12 months an intestinal microbiota which is dominated by Bifidobacteria such as *Bifidobacterium breve, Bifidobacterium infantis,* and *Bifidobacterium longum* to the exclusion of appreciable populations of such species as Clostridia and Streptococci and which is generally comparable with that found in a vaginally-delivered, breast fed infant of the same age.
"infant" means a child under the age of 12 months.
"prebiotic" means a non-digestible food ingredient that beneficially affects the host by selectively stimulating the growth and/or activity of one or a limited number of bacteria in the colon and thus improves host health (Gibson and Roberfroid "Dietary Modulation of the Human Colonic Microbiota: Introducing the Concept of Prebiotics" J. Nutr 125:1401 - 1412).
"probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

All references to percentages are percentages by weight unless otherwise stated.

*Bifidobacterium lactis* CNCM I-3446 is sold *inter alia* by the Christian Hansen company of Denmark under the trade mark Bb12. A suitable daily dose is from 10e5 to 10e11 colony forming units (cfu), more preferably from 10e7 to 10e10 cfu.

Preferably the *Bifidobacterium lactis* CNCM I-3446 is co-administered with a prebiotic: Suitable prebiotics include certain oligosaccharides, such as fructooligosaccharides (FOS) and galactooligosaccharides (GOS). A combination of prebiotics may be used such as 90% GOS with 10% short chain fructo-oligosaccharides such as the product sold under the trade mark Beneo® P95 or 10% inulin such as the product sold under the trade mark Beneo® HP, ST or HSI.

A particularly preferred prebiotic is an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc. Such an oligosaccharide mixture is described in more detail in WO2007/090894, the contents of which are incorporated herein by reference and is referred to hereinafter as "the oligosaccharide mixture described above".

Preferably the oligosaccharide mixture described above comprises 10-70 wt% of the specified N-acetylated oligosaccharide(s), 20-80 wt% of the specified neutral oligosaccharide(s) and 10-50 wt% of the specified sialylated oligosaccharide(s). More preferably the mixture comprises 15-40 wt% of the N-acetylated oligosaccharide(s), 40-60 wt% of the other neutral oligosaccharide(s) and 15-30 wt% of the sialylated oligosaccharide(s). A particularly preferred mixture is 30 wt% of the N-acetylated oligosaccharide(s), 50 wt% of the neutral oligosaccharide(s) and 20 wt% of the sialylated oligosaccharide(s).

Alternatively, the oligosaccharide mixture described above may conveniently comprise 5-20 wt% of the specified N-acetylated oligosaccharide(s), 60-90 wt% of the specified neutral oligosaccharide(s) and 5-30 wt% of the specified sialylated oligosaccharide(s)

The oligosaccharide mixture described above may be prepared from one or more animal milks. The milk may be obtained from any mammal, in particular from cows, goats, buffalos, horses, elephants, camels or sheep.

Alternatively the oligosaccharide mixture described above may be prepared by purchasing and mixing the individual components. For example, synthesised galacto-oligosaccharides such as Galβ1,6Galβ1,4Glc Galβ1,6Galβ1,6Glc, Galβ1,3Galβ1,4Glc, Galβ1,6Galβ1,6Galβ1,4Glc, Galβ1,6Galβ,3Galβ1,4Glc and Galβ1,3Galβ1,6Galβ1,4Glc and mixtures thereof are commercially available under the trade marks Vivinal ® and Elix'or ®. Other suppliers of oligosaccharides are Dextra Laboratories, Sigma-Aldrich Chemie GmbH and Kyowa Hakko Kogyo Co., Ltd. Alternatively, specific glycoslytransferases, such as galactosyltransferases may be used to produce neutral oligosaccharides.

The N-acetylated oligosaccharides may be prepared by the action of glucosaminidase and/or galactosaminidase on N-acetyl-glucose and/or N-acetyl galactose. Equally, N-acetyl-galactosyl transferases and/or N-acetyl-glycosyl transferases may be used for this purpose. The N-acetylated oligosaccharides may also be produced by fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerisation (DP) from DP=1 onwards. Another option is the chemical conversion of ketohexoses (e.g. fructose) either free or bound to an oligosaccharide (e.g. lactulose) into N-acetylhexosamine or an N-acetylhexosamine containing oligosaccharide as described in Wrodnigg, T.M.; Stutz, A.E. (1999) Angew. Chem. Int. Ed. 38:827-828.

The sialylated oligosaccharides 3'sialyl-lactose and 6'sialyl-lactose may be isolated by chromatographic or filtration technology from a natural source such as animal milks. Alternatively, they may also be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP) from DP=1 onwards.

Other probiotic bacteria may be administered with the *Bifidobacterium lactis* CNCM I-3446. Any lactic acid bacteria or Bifidobacteria with established probiotic characteristics may be used. Suitable probiotic lactic acid bacteria include *Lactobacillus rhamnosus* ATCC 53103 obtainable *inter alia* from Valio Oy of Finland under the trade mark LGG, *Lactobacillus rhamnosus* CGMCC 1.3724, *Lactobacillus reuteri* ATCC 55730 obtainable from Biogaia or *Lactobacillus paracasei* CNCM I-2116.

Suitable further probiotic Bifidobacteria strains include *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V and the strain of *Bifdobacterium breve* sold by Institut Rosell (Lallemand) under the trade mark R0070. A mixture of lactic acid bacteria and Bifidobacteria may be used.

The *Bifidobacterium lactis* CNCM I-3446 optionally with the oligosaccharide mixture described above is preferably administered to the infant immediately after delivery and thereafter for at least the first two months of the life of the infant. More preferably, administration continues until the infant reaches six months of age.

The *Bifidobacterium lactis* CNCM I-3446 may be administered directly to the infant or, if the mother is breast-feeding, via the mother. If administration is to be via the mother, this may be as a supplement in the form of tablets, capsules, pastilles, chewing gum or a liquid for example. The supplement preferably also contains the oligosaccharide mixture described above in an amount of from 0.2 to 10g/day. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The supplement may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

Alternatively, the *Bifidobacterium lactis* CNCM I-3446 may be administered to the mother in the form of a therapeutic nutritional composition. The composition may be a nutritionally complete formula.

A nutritionally complete formula for administration to lactating women according to the invention may comprise a source of protein. Any suitable dietary protein may be used for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. The composition may also contain a source of carbohydrates and a source of fat.

If the formula includes a fat source in addition to the DHA, the fat source preferably provides 5% to 40% of the energy of the formula; for example 20% to 30% of the energy. A suitable fat profile may be obtained using a blend of canola oil, corn oil and high-oleic acid sunflower oil.

A source of carbohydrate may be added to the formula. It preferably provides 40% to 80% of the energy of the formula. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, partially hydrolysed guar gum, gum Arabic, fructooligosaccharides and galacto-oligosaccharides. Preferably, if fibre is present, the fibre content is between 2 and 40 g/l of the formula as consumed, more preferably between 4 and 10 g/l. In addition, the formula also preferably contains the oligosaccharide mixture described above in an amount of from 0.2 to 5 grams per litre of reconstituted formula, preferably 1 to 2 g/l.

The formula may also contain minerals and micronutrients such as trace elements and vitamins in accordance with the recommendations of Government bodies such as the USRDA. For example, the formula may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B 12, 100 to 800 µg folic acid, 30 to 70 µg biotin, 1 to 5 µg Vitamin D, 3 to 10 IU Vitamin E.

One or more food grade emulsifiers may be incorporated into the formula if desired; for example diacetyl tartaric acid esters of mono- and di- glycerides, lecithin and mono- and di-glycerides. Similarly suitable salts and stabilisers may be included.

The formula is preferably enterally administrable; for example in the form of a powder-for re-constitution with milk or water.

Alternatively, or in the case of infants who are not breast fed, the *Bifidobacterium lactis* CNCM I-3446 may be administered to the infant as a supplement, for example as a daily dose of 10e10 cfu dissolved in water and administered on a spoon.

For infants who are not breast fed, the *Bifidobacterium lactis* CNCM I-3446 may be conveniently administered in an infant formula.

An infant formula for use according to the present invention may contain a protein source in an amount of not more than 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The infant formula may contain a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

The infant formula may contain a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like.

Preferably, the infant formula will contain the oligosaccharide mixture described above in an amount of from 0.2 to 5 grams per litre of reconstituted formula, preferably 1 to 2 g/l.

The infant formula may optionally contain other substances which may have a beneficial effect such as lactoferrin, nucleotides, nucleosides, and the like.

Both the infant formula and the nutritional formula described above may be prepared in any suitable manner. For example, they may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight.

The *Bifidobacterium lactis* CNCM I-3446 may be cultured according to any suitable method and prepared for addition to the nutritional or infant formula by freeze-drying or spray-drying for example. Alternatively, *Bifidobacterium lactis* CNCM I-3446 can be bought from Christian Hansen under the trade mark Bb12®-already prepared in a suitable form for addition to food products such as nutritional and infant formulas. The *Bifidobacterium lactis* CNCM I-3446 may be added to the formula in an amount between 10e3 and 10e12 cfu/g powder, more preferably between 10e7 and 10e12 cfu/g powder.

The invention will now be further illustrated by reference to the following examples:-

### Example 1

An example of the composition of a suitable infant formula to be used in the present invention is given below

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *Bifidobacterium lactis* CNCM I-3446 | 2.10⁷ cfu/g of powder, live bacteria | |

### Example 2

This example compares the effect of *Bifidobacterium lactis* CNCM I-3446 with and without the addition of an oligosaccharide ingredient including N-acetylated oligosaccharides, neutral oligosaccharides and sialylated oligosaccharides (referred to hereinafter as CMOS-GOS) on the establishment of an early bifidogenic intestinal microbiota in a gnotobiotic mouse model of caesarean delivery with the effect of another strain of Bifidobacteria and with a control. This model is an appropriate animal model of infants born by caesarean delivery and having a sub-optimal intestinal microbiota in terms of population of Bifidobacteria. In addition to the observation of the size of Bifidobacteria population, this model is also suitable to follow the beneficial effect of the Bifidobacteria as a barrier against potentially pathogenic bacteria like *Clostridium perfringens.*

### Materials and Methods

Germfree C3H female and male mice were purchased from Charles River Laboratories France and shipped to the Nestle Research Centre in transportation isolators. Animals were transferred to breeding isolators after control of germfree status. Female offspring of this breeding population was used for this study. After weaning animals were randomly assigned to one of the 4 study groups: A, control diet and control drink; B, control diet and probiotic *B. lactis* CNCM I-3446 drink; C, prebiotic CMOS-GOS diet and probiotic *B. lactis* CNCM I-3446 drink; D, control diet and probiotic *B. longum* ATCC BAA-999 drink.

Animals were kept in different isolators in cages of 5 animals each. Group A was kept in one isolator, Groups B and C were kept in a second isolator and Group D was kept in a third isolator. Germfree status was monitored weekly in freshly collected faeces from one animal per cage. During this period, the animals were fed the diet AIN-93 basal (see Table 1 below).

At the age of 7 to 8 weeks 2 animals per cage were re-controlled for germfree status and each animal received thereafter by gavage a single dose of 200 µl human baby microbiota cocktail (HBF) as described in Table 2 below. After gavage all animals remained on the basal diet for 2 weeks to allow for establishment of the HBF in the intestine. Then the solid diet was changed to the AIN-mix (Table 1) (for groups A, B and D) or to the AIN-CMOS-GOS (for group C). Drinking water was changed to saline drinking water containing 0.5 % (v/v) MRS (Man Rogosa Sharpe) (for group A) or saline drinking water containing probiotic bacteria and 0.5 % (v/v) MRS (for groups B, C and D). The final concentrations of probiotics were 2.5 × 10e7 cfu/ml *B. lactis* CNCM I-3446 (for groups B and C) and 2.2 × 10e7 cfu/ml *B. longum* ATCC BAA-999 (for group D).

*Bifidobacterium lactis* CNCM I3446 was prepared from the Nestle culture collection. Briefly, strains were reactivated and grown in MRS medium to about 1.5 × 10e9 cfu/ml. Thereafter the strain was concentrated by centrifugation in its spent MRS medium and diluted to 4.9 × 10e9 cfu/ml with fresh MRS medium. It was then divided in 1 ml aliquots that were frozen at -80°C until used. Each day one freshly defrosted 1 ml aliquot of *Bifidobacterium lactis* CNCM I3446 in MRS (for groups B and C) or 1 ml MRS (for group A) was introduced in the isolators, dissolved in 200 ml saline and divided equally between drinking bottles. With an average consumption of 5 ml/day and mouse each animal of the groups with *B. lactis* received about 10e8 cfu *B. lactis* per day.

*Bifidobacterium longum* ATCC BAA-999 was prepared from the Nestle culture collection. Briefly, strains were reactivated and grown in MRS medium to about 1 x 10e9 cfu/ml. Thereafter the strain was concentrated by centrifugation and diluted to 4.4 × 10e9 cfu/ml with fresh MRS medium. It was then divided in 1 ml aliquots that were frozen at -80°C until used. Each day one freshly defrosted 1 ml aliquot of *Bifidobacterium longum* ATCC BAA-999 in MRS (for group D) was introduced in the isolators, dissolved in 200 ml saline and divided equally to drinking bottles. With an average consumption of 5 ml/day and mouse each animal of the group with *B longum* received about 10e8 cfu *B longum* per day.

The oligosaccharide ingredient CMOS-GOS was prepared starting from an industrial deproteinated and demineralized whey permeate (Lactosérum France, France). Briefly, an ultrafiltration cow milk whey permeate was demineralized on an industrial demineralization line equipped with electrodialysis modules and, anion- and cation exchangers (Lactosérum France). The demineralized whey permeate was then subjected to 2 sequential industrial lactose crystallisation cycles and was subsequently spray dried (Lactosérum France). The resulting powdered modified mother liquor was hydrated in an atmosphere with elevated relative humidity (ca. 43%) established by a saturated solution of K₂CO₃ in a closed container. This allowed for the formation of crystalline lactose. Cold water was added to the rehydrated powder (about 2 -3 liters per kg of rehydrated powder) and centrifuged at 10'000 x g for 20 minutes. The supernatant was collected and the pellet suspended again with cold water and centrifuged as before. The second supernatant was pooled with the first one and both were lyophilized. This lactose-reduced cows' milk oligosaccharide ingredient (CMOS) was analysed by high performance anion exchange chromatography system equipped with pulsed amperometric detection (HPAEC-PAD; ICS3000, Dionex, Sunnyvale, CA) using a CarboPac PA200 (Dionex) analytical column equipped with a CarboPac amino trap column guard (Dionex). The CMOS preparation contained the original oligosaccharides and about 3 % (w/w) glucose, 46 % (w/w) lactose, 0.84 % (w/w) sialyllactose. Galactosyloligosaccharides (Vivinal GOS 259) were purchased from Friesland Foods DOMO. The ingredient is sold as syrup composed of ca. 75% dry matter (DM) of which are lactose 23 % (on DM), glucose 22 % (on DM), galactosyloligosaccharides 59 % (on DM) and were mixed with the CMOS preparation to obtain a CMOS-GOS ingredient containing about 9 wt % N-acetylated oligosaccharides, about 82 wt % neutral oligosaccharides and about 9 wt % sialylated oligosaccharides.

Mice were exclusively fed a semi-synthetic AIN-93 diet and modifications thereof (Table 1). From day 15 post-partum onwards mice were fed AIN-93 basal diet. At the start of the intervention at around 8 weeks of age mice were fed for 14 days with 'AIN-mix' (groups A, B and D) or 'AIN-CMOS-GOS' (group C).

**Table 1. Composition of the used AIN diets in g/100g of diet**

| | **AIN-93 basal** | **AIN mix** | **AIN-CMOS-GOS** |
|---|---|---|---|
| Corn starch | 51.5 | 49.8 | 21.5 |
| Cellulose | 5 | 5 | 5 |
| Sucrose | 10 | 10 | 10 |
| Glucose | - | 1.45 | 1.45 |
| Lactose | - | 12.3 | 12.3 |
| CMOS GOS² | - | - | 2.3 |
| Casein | 20 | 20 | 20 |
| Soybean oil | 7 | 7 | 7 |
| Mineral mix AIN93G | 3.5 | 3.5 | 3.5 |
| Vitamin mix AIN93 ¹ | 2.5 | 2.5 | 2.5 |
| Choline bitartrate | 0.25 | 0.25 | 0.25 |
| L-cystine | 0.3 | 0.3 | 0.3 |
| Tert-butylhydroquinone | 0.0014 | 0.0014 | 0.0014 |

| | | | |
|---|---|---|---|
| ¹ Vitamin mix was supplemented with vitamin B1 (thiamine-HCl) at 330 mg/kg vitamin mix to reach required levels of 600 mg/kg. ² Including SL at 0.2 g/100g. | | | |

**Table 2 - Microbiota Composition**

| **strain** | **Colony phenotype on plate** | **concentration administred log(cfu/ml)** |
|---|---|---|
| *Bifidobacterium breve* NCC452 (viv4) | white, big | 8.85 |
| *Bifidobacterium longum* NCC572 (viv5) | grey, small | 8.19 |
| *Staphylococcus aureus* FSM124 (viv3) | | 8.48 |
| *Staphylococcus epidermidis* FSM115 (viv2) | | 8.48 |
| *Escherichia coli* FSM325 (viv1) | | 8.48 |
| *Bacteroides distasonis* FSM24 (viv20) | | 8.48 |
| *Clostridium perfringens* FSM-C14 (viv19) | | 6.0 |

Verification of germfree status was done using single freshly collected faeces 1 to 2 per cage. Briefly, one freshly collected faecal pellet was homogenized in 0.5 mL Ringer solution (Oxoid, UK) supplemented with 0.05 % (w/v) L-Cystein (HCl) and 2 times 100 ul thereof were plated on 2 TSS plates (Trypcase soy agar with 5% sheep blood; BioMerieux, France). One plate was incubated aerobic for 24 h at 37°C and the second plate was incubated anaerobic for 48 h at 37°C.

Faecal samples were collected and analysed on day 14. Briefly, for each mouse 1 faecal pellet was homogenized in 0.5 mL Ringer solution (Oxoid, UK) supplemented with 0.05 % (w/v) L-Cysteine (HCl) and different dilution of the bacterial solution were plated on selective and semi-selective media for the enumeration of specific micro-organisms: Bifidobacteria on Eugom Tomato medium, Lactobacillus on MRS medium supplemented with antibiotics (phosphomycine, sulfamethoxazole and trimethoprime), C. perfringens on NN-agar medium, Enterobacteriaceae on Drigalski medium, and Bacteroides on Shaedler Neo Vanco medium. Plates were incubated at 37 °C under aerobic conditions for 24 h for the counting of Enterobacteriaceae, and under anaerobic conditions during 48 h for Bifidobacteria, Lactobacillus, Bacteroides and C. perfringens.

On day 14 animals were sacrificed. Briefly, 2 cages per isolator were simultaneously removed of a given isolator, while keeping the isolator germfree until all animals were removed. Each animal was weighed. Immediately thereafter the animal was euthanized by decapitation and exhaustive bleeding. Blood was collected and animals were dissected to collect a jejunum sample (about 6-7 cm directly after duodenum) for microbiota analysis.

### Results

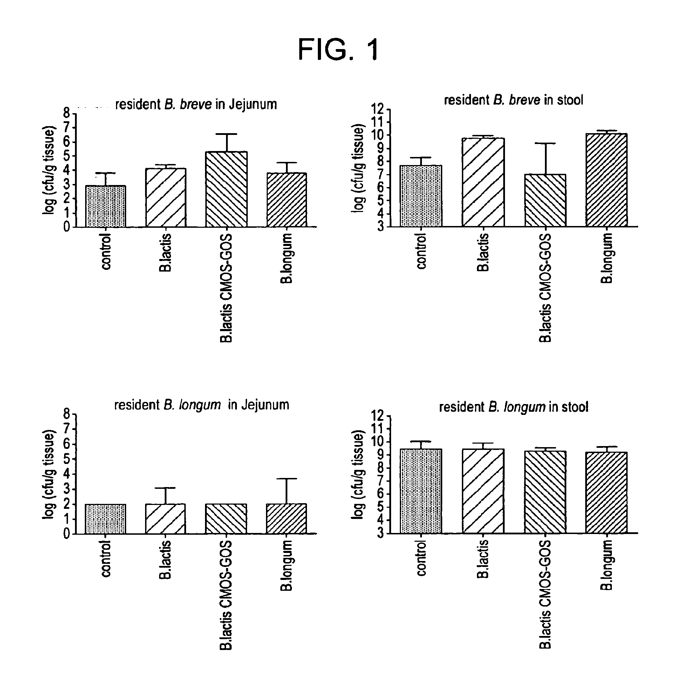

Figure 1 depicts the counts of the two human baby microbiota resident Bifidobacteria - *B. breve* and *B. longum -* in the small intestine (jejunum) and faeces after two weeks of treatment. In the jejunum, *B. breve* counts were promoted in groups B and C. In stools, increased counts were found in groups B and D. No significant changes of *B. longum* counts were found between the groups.

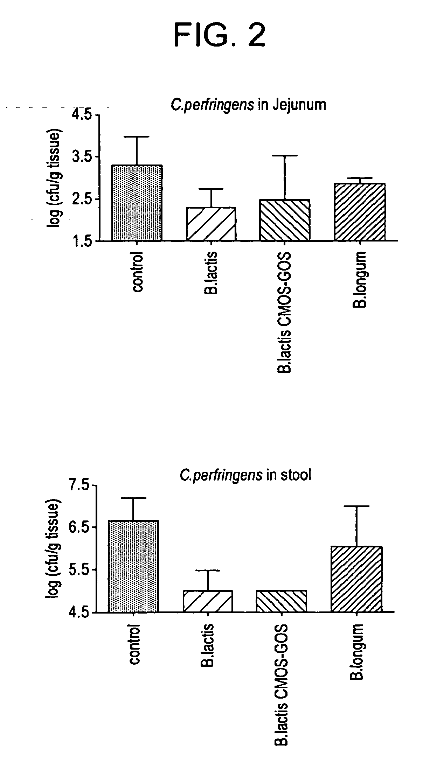

Counts of *C. perfringens* in the small intestine (jejunum) and faeces after two weeks of treatment are shown in Figure 2. In the jejunum, reduced levels of *C*. *perfringens* counts are seen for Groups B and C with *B. lactis* but not for groups A and D without *B. lactis.* This effect is even more marked for the stool samples.

Levels of *B. lactis* above the threshold (10e5 cfu/g faeces) were recovered from about 50% of groups B and C after 1 week, but from none of them after 2 weeks. As may be seen from Table 3 below, despite the relative high daily dose administered *B. lactis* did not out-compete the resident Bifidobacteria and remained a minor constituent of the microbiota.

**Table 3. B. lactis counts in faeces and jejunum content over time of treatment.**

| Group | > 10⁵ cfu / g faeces | | > 10² cfu / g jejunum |
|---|---|---|---|
| | week1 | week2 | week2 (jejunum) |
| B | 5/9 | 0/9 | 3/9 |
| C | 3/9 | 0/9 | 6/9 |

## Claims

1. The use of *Bifidobacterium lactis* CNCM I-3446 in the manufacture of a medicament or therapeutic nutritional composition for promoting the development of an early bifidogenic intestinal microbiota in infants delivered by caesarean section.

2. The use of *Bifidobacterium lactis* CNCM I-3446 in the manufacture of a medicament or therapeutic nutritional composition for reducing the risk of subsequent development of allergy in infants delivered by caesarean section.

3. The use of *Bifidobacterium lactis* CNCM I-3446 in the manufacture of a medicament or therapeutic nutritional composition for preventing or treating diarrhoea in infants delivered by caesarean section

4. The use of any of Claims 1 to 3 wherein the medicament or therapeutic nutritional composition further comprises an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc.

5. The use of Claim 4, wherein the oligosaccharide mixture comprises 10-70 wt% of the N-acetylated oligosaccharides, 20-80 wt% of the neutral oligosaccharides and 10-50 wt% of the sialylated oligosaccharides.

6. The use of Claim 4 or 5 wherein the oligosaccharide mixture comprises 15-40 wt% of the N-acetylated oligosaccharides, 40-60 wt% of the neutral oligosaccharides and 15-30 wt% of the sialylated oligosaccharides.

7. The use of Claim 4 or 5, wherein the oligosaccharide mixture comprises 5-20 wt% of the N-acetylated oligosaccharides, 60-90 wt% of the neutral oligosaccharides and 5-30 wt% of the sialylated oligosaccharides.

8. The use of any preceding claim wherein the medicament or therapeutic nutritional composition is administered to the infant immediately after delivery and thereafter for at least 2 months.

9. The use of any preceding claim, wherein the medicament or therapeutic nutritional composition is administered to the infant for at least 6 months after delivery.

10. The use of any preceding claim, wherein the *Bifidobacterium lactis* CNCM I-3446 is administered to the infant via the breast-feeding mother.

11. The use of any of Claims 1 to 9, wherein the therapeutic nutritional composition is an infant formula.

12. The use of any preceding claim wherein the medicament comprises between 10e5 and 10e11 cfu of *Bifidobacterium lactis* CNCM I-3446 per daily dose.

13. The use of any of Claims 1 to 11 wherein the therapeutic nutritional composition comprises between 10e3 and 10e12 cfu of *Bifidobacterium lactis* CNCM I-3446 per gram of composition (dry weight).

## Patentansprüche

1. Verwendung von *Bifidobacterium lactis* CNCM 1-3446 bei der Herstellung eines Medikamentes oder einer therapeutischen Nahrungszusammensetzung zur Förderung der Entwicklung einer frühen bifidogenen Darmmikrobiota bei Säuglingen, die durch Kaiserschnitt geboren wurden.

2. Verwendung von *Bifidobacterium lactis* CNCM 1-3446 bei der Herstellung eines Medikamentes oder einer therapeutischen Nahrungszusammensetzung zur Reduzierung der Gefahr einer anschließenden Entwicklung einer Allergie bei Säuglingen, die durch Kaiserschnitt geboren wurden.

3. Verwendung von *Bifidobacterium lactis* CNCM 1-3446 bei der Herstellung eines Medikamentes oder einer therapeutischen Nahrungszusammensetzung zur Vorbeugung oder Behandlung von Durchfall bei Säuglingen, die durch Kaiserschnitt geboren wurden.

4. Verwendung nach einem der Ansprüche 1 bis 3, wobei das Medikament oder die therapeutische Nahrungszusammensetzung weiterhin eine Oligosaccharidmischung aufweist, die 5-70 Gew.-% von mindestens einem N-acetylierten Oligosaccharid aufweist, das aus der Gruppe mit GalNAcαI,3GalβI,4Glc und GalβI,6GalNAcαI,3GalβI,4Glc, ausgewählt ist, 20-90 Gew.-% von mindestens einem neutralen Oligosaccharid aufweist, das aus der Gruppe mit GalβI,6Gal,GalβI,6GalβI,4Glc, GalβI,6GalβI,6Glc, Galβl,3Galβl,3Glc,Galβl,3Galβl,4Glc, Galβl,6Galβl,6Galβl,4Glc,Galβl,6Galβl,3Galβl,4Glc,Galβl,3Galβl,6Galβl,4Glc und GalβI,3GalβI,3GalβI,4Glc ausgewählt ist, und 5-50 Gew.-% von mindestens einem sialylierten Oligosaccharid aufweist, das aus der Gruppe mit NeuAcα2,3GalβI,4Glc und NeuAcα2,6GalβI,4Glc ausgewählt ist.

5. Verwendung nach Anspruch 4, wobei die Oligosaccharidmischung 10-70 Gew.-% der N-acetylierten Oligosaccharide, 20-80 Gew.-% der neutralen Oligosaccharide und 10-50 Gew.-% der sialylierten Oligosaccharide aufweist.

6. Verwendung nach Anspruch 4 oder 5, wobei die Oligosaccharidmischung 15-40 Gew.-% der N-acetylierten Oligosaccharide, 40-60 Gew.-% der neutralen Oligosaccharide und 15-30 Gew.-% der sialylierten Oligosaccharide aufweist.

7. Verwendung nach Anspruch 4 oder 5, wobei die Oligosaccharidmischung 5-20 Gew.-% der N-acetylierten Oligosaccharide, 60-90 Gew.-% der neutralen Oligosaccharide und 5-30 Gew.-% der sialylierten Oligosaccharide aufweist.

8. Verwendung nach einem der vorhergehenden Ansprüche, wobei das Medikament oder die therapeutische Nahrungszusammensetzung dem Säugling unmittelbar nach der Geburt und danach mindestens zwei Monate lang verabreicht wird.

9. Verwendung nach einem der vorhergehenden Ansprüche, wobei das Medikament oder die therapeutische Nahrungszusammensetzung dem Säugling nach der Geburt mindestens sechs Monate lang verabreicht wird.

10. Verwendung nach einem der vorhergehenden Ansprüche, wobei das *Bifidobacterium lactis* CNCM 1-3446 dem Säuglingen über die stillende Mutter verabreicht wird.

11. Verwendung nach Anspruch 1 bis 9, wobei die therapeutische Nahrungszusammensetzung eine Säuglingsnahrung ist.

12. Verwendung nach einem der vorhergehenden Ansprüche, wobei das Medikament zwischen 10e5 und 10e11 cfu von *Bifidobacterium lactis* CNCM 1-3446 pro Tagesdosis aufweist.

13. Verwendung nach einem der Ansprüche 1 bis 11, wobei die therapeutische Nahrungszusammensetzung zwischen 10e3 und 10e12 cfu von *Bifidobacterium lactis* CNCM 1-3446 pro Gramm der Zusammensetzung (Trockengewicht) aufweist.

## Revendications

1. Utilisation de *Bifidobacterium lactis* CNCM I-3446 dans la fabrication d'un médicament ou d'une composition nutritionnelle thérapeutique pour promouvoir le développement d'un premier microbiote intestinal bifidogène chez les nourrissons nés par césarienne.

2. Utilisation de *Bifidobacterium lactis* CNCM I-3446 dans la fabrication d'un médicament ou d'une composition nutritionnelle thérapeutique pour réduire le risque de développement ultérieur d'allergies chez les nourrissons nés par césarienne.

3. Utilisation de *Bifidobacterium lactis* CNCM I-3446 dans la fabrication d'un médicament ou d'une composition nutritionnelle thérapeutique pour prévenir ou traiter la diarrhée chez les nourrissons nés par césarienne.

4. Utilisation d'une des revendications de 1 à 3 où le médicament ou la composition nutritionnelle thérapeutique comprend en outre un mélange d'oligosaccharides qui comprend 5-70 wt% d'au moins un oligosaccharide N-acétylé sélectionné dans le groupe comprenant GalNAcα1,3Galβ1,4Glc et Galβ1,6GalNAcα1,3Galβ1,4Glc, 20-90 wt% d'au moins un oligosaccharide neutre sélectionné dans le groupe comprenant Galβ1,6Gal, Galβ1,6Galp1,4Glc
Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Gaβ1,4Glc Galβ1,3Galβ1,6Galβ1,4Glc et Galβ1,3Galβ1,3Galβ1,4Glc et 5-50 wt% d'au moins un oligosaccharide sialylé sélectionné dans le groupe comprenant NeuAcα2,3Galβ1,4Glc et NeuAcα2,6Galβ,4Glc.

5. Utilisation de la revendication 4 où le mélange d'oligosaccharides comprend 10-70 wt% d'oligosaccharides N-acétylés, 20-80 wt% d'oligosaccharides neutres et 10-50 wt% d'oligosaccharides sialylés.

6. Utilisation des revendications 4 ou 5 où le mélange d'oligosaccharides comprend 15-40 wt% d'oligosaccharides N-acétylés, 40-60 wt% d'oligosaccharides neutres et 15-30 wt% d'oligosaccharides sialylés.

7. Utilisation des revendications 4 ou 5 où le mélange d'oligosaccharides comprend 5-20 wt% d'oligosaccharides N-acétylés, 60-90 wt% d'oligosaccharides neutres et 5-30 wt% d'oligosaccharides sialylés.

8. Utilisation de l'une des revendications précédentes où le médicament ou la composition nutritionnelle thérapeutique est administré au nourrisson immédiatement après l'accouchement et par la suite pendant 2 mois minimum.

9. Utilisation de l'une des revendications précédentes où le médicament ou la composition nutritionnelle thérapeutique est administré au nourrisson pendant au moins 6 mois après l'accouchement.

10. Utilisation de l'une des revendications précédentes où le *Bifidobacterium lactis* CNCM I-3446 est administré au nourrisson par allaitement.

11. Utilisation de l'une des revendications de 1 à 9 où la composition nutritionnelle thérapeutique est une préparation pour nourrisson.

12. Utilisation de l'une des précédentes revendications où le médicament comprend entre 10e5 et 10e11 cfu de *Bifidobacterium lactis* CNCM I-3446 par dose quotidienne.

13. Utilisation de l'une des revendications de 1 à 11 où la composition nutritionnelle thérapeutique comprend entre 10e3 et 10e12 cfu de *Bifidobacterium lactis* CNCM I-3446 par gramme de composition (poids sec).
